# EUROPEAN PATENT APPLICATION

(11) **EP 4 830 667 A1**
(43) Date of publication of application: **28.10.2026**
(21) Application number: 24916608.3
(22) Date of filing: 02.12.2024
(51) Int. Cl.: A61F 2/24

(54) **VALVE STENT, METHOD FOR PROCESSING VALVE STENT, AND ARTIFICIAL VALVE**

(30) Priority: 11.01.2024 CN 202410039309
(71) Applicant: Beijing Med-Zenith Medical Scientific Corporation Limited, Tianzhu Free Trade Zone Shunyi District Beijing 101312 (CN)
(72) Inventor: GAO, Xin, Beijing 101312 (CN); MA, Zhiwei, Beijing 101312 (CN); ZHANG, Haoran, Beijing 101312 (CN); LIU, Yan, Beijing 101312 (CN); SHI, Huanhuan, Beijing 101312 (CN); ZHOU, Qingliang, Beijing 101312 (CN); MENG, Jian, Beijing 101312 (CN)
(74) Representative: Huang, Liwei
(86) International application number: PCT/CN2024/136033
(87) International publication number: WO 2025/148560

(57) **Abstract**

The present disclosure provides a valve stent, a method for forming the valve stent, and an artificial heart valve. The valve stent is formed by cutting metal tubing, has a post-cutting state, and includes a plurality of grid structure units; each grid structure unit includes a plurality of quadrilateral grid structure, and the plurality of quadrilateral grid structures each include a middle supporting grid structure; and in the post-cutting state, an included angle formed by straight segments of two stent struts that are arranged opposite each other in an circumferential direction of the valve stent in the middle supporting grid structure is less than or equal to 12°.

## Description

### CROSS-REFERENCE TO RELATED APPLICATIONS

The present application claims priority to Chinese Patent Application No. 202410039309.5, filed on Jan. 11, 2024 and entitled "VALVE STENT, METHOD FOR FORMING VALVE STENT, AND ARTIFICIAL HEART VALVE", which is incorporated herein by reference in its entirety.

### TECHNICAL FIELD

The present application relates to the field of medical devices, and in particular, to a valve stent, a method for forming the valve stent, and an artificial heart valve.

### BACKGROUND

In the related art, valve stents of some artificial heart valves are formed by cutting metal tubing.

A valve stent formed by metal cutting is integrally manufactured from the same material and using the same manufacturing process, and such a valve stent is susceptible to fracture during processing and also presents issues relating to safety and clinical effectiveness during use.

The foregoing description is provided merely for providing background technical information related to the present disclosure and does not necessarily constitute prior art.

### SUMMARY

An objective of the present disclosure is to provide a valve stent, a method for forming the valve stent, and an artificial heart valve, so as to alleviate or solve the problems that a valve stent formed by cutting metal tubing is susceptible to fracture during processing and presents issues relating to safety and clinical effectiveness during use.

According to a first aspect, the present disclosure provides a valve stent. The valve stent is formed by cutting metal tubing and has a post-cutting state and an expanded state, the valve stent includes a plurality of grid structure units distributed along a circumferential direction of the valve stent, and the plurality of grid structure units extend along an axial direction of the valve stent; each grid structure unit includes a plurality of quadrilateral grid structure, the plurality of quadrilateral grid structures include at least one near-end grid structure, at least one middle supporting grid structure, and at least one far-end grid structure sequentially arranged along the axial direction of the valve stent from a first axial end to a second axial end; each quadrilateral grid structure includes four stent struts connected end to end in sequence, each stent strut includes a straight segment and a connecting segment, the straight segments of the stent struts form a grid body of the quadrilateral grid structure, the connecting segments of the stent struts form corner portions of the quadrilateral grid structure, adjacent stent struts of each quadrilateral grid structure are connected through the connecting segment, and corner portions at an axial middle portion of a corresponding quadrilateral grid structure of adjacent grid structure units are connected; and when the valve stent is in the post-cutting state, straight segments of two stent struts that are arranged opposite each other in the circumferential direction of the valve stent in each quadrilateral grid structure gradually diverge from each other from the axial middle portion toward an axial end portion of the corresponding quadrilateral grid structure to form an included angle, where an included angle of the middle supporting grid structure formed by the straight segments of the two stent struts that are arranged opposite each other in the circumferential direction of the valve stent is less than or equal to 12°.

In the valve stent according to some embodiments,
the plurality of quadrilateral grid structures include two middle supporting grid structures, and the included angle of each middle supporting grid structure formed by the straight segments of the two stent struts that are arranged opposite each other in the circumferential direction of the valve stent is less than 11.74°; or
the plurality of quadrilateral grid structures include one middle supporting grid structure, and the included angle of the middle supporting grid structure formed by the straight segments of the two stent struts that are arranged opposite each other in the circumferential direction of the valve stent is less than 6.14°.

In the valve stent according to some embodiments,
the plurality of quadrilateral grid structures include two middle supporting grid structures, and the included angle of each middle supporting grid structure formed by the straight segments of the two stent strut that are arranged opposite each other in the circumferential direction of the valve stent is 5.0° to 11.0°; or
the plurality of quadrilateral grid structures include one middle supporting grid structure, and the included angle of the middle supporting grid structure formed by the straight segments of the two stent struts that are arranged opposite each other in the circumferential direction of the valve stent is 2.5° to 5.5°.

In the valve stent according to some embodiments, when the valve stent is in the post-cutting state, an included angle of the middle supporting grid structure formed by the straight segments of the two stent struts adjacent to the first axial end is equal to or different from an included angle of the middle supporting grid structure formed by the straight segments of the two stent struts adjacent to the second axial end.

In the valve stent according to some embodiments, the connecting segment of the stent strut includes two curved connecting segments respectively provided at two ends of the straight segment, and two adjacent stent struts in the quadrilateral grid structure are connected through the curved connecting segments.
a curved connecting segment of the stent strut adjacent to an axial end portion of a quadrilateral grid structure in which the stent strut is located is curved toward an inner side of the quadrilateral grid structure; and
a curved connecting segment of the stent strut adjacent to an axial middle portion of the quadrilateral grid structure in which the stent strut is located is curved toward an outer side of the quadrilateral grid structure.

In the valve stent according to some embodiments, the curved connecting segment is an arcuate connecting segment, and a ratio of a length of the straight segment to an inner radius of the arcuate connecting segment is in a range of 4 to 30.

In the valve stent according to some embodiments, the straight segment is connected to the curved connecting segment through a curved transition segment, and a bending direction of the curved transition segment is opposite to a bending direction of the curved connecting segment.

In the valve stent according to some embodiments, the curved transition segment is an arcuate transition segment, and a ratio of an inner radius of the arcuate transition segment to the length of the straight segment is in a range of 0.25 to 0.75.

In the valve stent according to some embodiments, the curved connecting segment is an arcuate connecting segment, where
a length of the near-end grid structure along the axial direction of the valve stent between centers of arcuate connecting segments at two ends of a stent strut adjacent to the first axial end is equal to or different from a length of the near-end grid structure along the axial direction of the valve stent between the centers of the arcuate connecting segments at two ends of the stent strut adjacent to the second axial end; and/or
a length of the far-end grid structure along the axial direction of the valve stent between the centers of the arcuate connecting segments at two ends of the stent strut adjacent to the first axial end is equal to or different from a length of the far-end grid structure along the axial direction of the valve stent between the centers of the arcuate connecting segments at two ends of the stent strut adjacent to the second axial end; and/or
a length of the middle supporting grid structure along the axial direction of the valve stent between the centers of the arcuate connecting segments at two ends of the stent strut adjacent to the first axial end is equal to or different from a length of the middle supporting grid structure along the axial direction of the valve stent between the centers of the arcuate connecting segments at two ends of the stent strut adjacent to the second axial end.

In the valve stent according to some embodiments, the length of the near-end grid structure along the axial direction of the valve stent between the centers of the arcuate connecting segments at two ends of the stent strut adjacent to the first axial end is less than the length of the near-end grid structure along the axial direction of the valve stent between the centers of the arcuate connecting segments at two ends of the stent strut adjacent to the second axial end; and/or
the length of the far-end grid structure along the axial direction of the valve stent between the centers of the arcuate connecting segments at two ends of the stent strut adjacent to the first axial end is greater than the length of the far-end grid structure along the axial direction of the valve stent between the centers of the arcuate connecting segments at two ends of the stent strut adjacent to the second axial end.

In the valve stent according to some embodiments, a ratio of the length of the near-end grid structure along the axial direction of the valve stent between the centers of the arcuate connecting segments at two ends of the stent strut adjacent to the first axial end and the length of the near-end grid structure along the axial direction of the valve stent between the centers of the arcuate connecting segments at two ends of the stent strut adjacent to the second axial end is 0.65 to 1.00; and/or
a ratio of the length of the far-end grid structure along the axial direction of the valve stent between the centers of the arcuate connecting segments at two ends of the stent strut adjacent to the second axial end and the length of the far-end grid structure along the axial direction of the valve stent between the centers of the arcuate connecting segments at two ends of the stent strut adjacent to the first axial end is 0.65 to 1.00.

In the valve stent according to some embodiments,
the curved connecting segment is an arcuate connecting segment;
the plurality of quadrilateral grid structures include one near-end grid structure, one middle supporting grid structure, and one far-end grid structure sequentially arranged along the axial direction of the valve stent from the first axial end to the second axial end;
a length of the middle supporting grid structure along the axial direction of the valve stent between centers of arcuate connecting segments at two ends of each stent strut is in a range of 3.05 mm to 8.22 mm; and/or
a length of the near-end grid structure along the axial direction of the valve stent between the centers of the arcuate connecting segments at two ends of each stent strut is in a range of 3.55 mm to 12.50 mm; and/or
a length of the far-end grid structure along the axial direction of the valve stent between the centers of the arcuate connecting segments at two ends of each stent strut is in a range of 3.55 mm to 12.50 mm.

In the valve stent according to some embodiments,
the curved connecting segment is an arcuate connecting segment;
for each grid structure unit,
${L}_{i} \times tan \frac{{α}_{i}}{2} = {L}_{j} \times tan \frac{{α}_{j}}{2}$
where
Lᵢ is a length of the quadrilateral grid structure along the axial direction of the valve stent between centers of arcuate connecting segments at two ends of a stent strut adjacent to the first axial end;
Lⱼ is a length of the quadrilateral grid structure along the axial direction of the valve stent between the centers of the arcuate connecting segments at two ends of the stent strut adjacent to the second axial end;
αᵢ is an included angle of the quadrilateral grid structure formed by the straight segments of the two stent struts adjacent to the first axial end; and
αⱼ is an included angle of the quadrilateral grid structure formed by the straight segments of the two stent struts adjacent to the second axial end.

In the valve stent according to some embodiments, the valve stent further includes a plurality of connecting rods, where
the near-end grid structure and the middle supporting grid structure of each grid structure unit are connected through one connecting rod; or, along the circumferential direction of the valve stent, one grid structure unit in which the near-end grid structure and the middle supporting grid structure are connected through one connecting rod is provided for every one or two grid structure units in which the near-end grid structure is not connected to the middle supporting grid structure through the connecting rod; and/or
the far-end grid structure and the middle supporting grid structure of each grid structure unit are connected through one connecting rod; or, along the circumferential direction of the valve stent, one grid structure unit in which the far-end grid structure and the middle supporting grid structure are connected through one connecting rod is provided for every one or two grid structure units in which the far-end grid structure is not connected to the middle supporting grid structure through the connecting rod.

In the valve stent according to some embodiments, when the valve stent is in the expanded state, an outer diameter D₂ at the first axial end and the second axial end of the valve stent is greater than an outer diameter D₁ at the axial middle portion.

In the valve stent according to some embodiments, when the valve stent is in the expanded state, the outer diameter D₁ at the axial middle portion of the valve stent is in a range of 18.0 mm to 32.0 mm, and the outer diameter D₂ at the first axial end and the second axial end of the valve stent is in a range of 20.0 mm to 54.0 mm.

In the valve stent according to some embodiments, when the valve stent is in the expanded state, a total height H₁ of the valve stent is in a range of 30.0 mm to 60.0 mm, and a height H₂ of the middle supporting grid structure is in a range of 12.0 mm to 30.0 mm.

In the valve stent according to some embodiments, when the valve stent is in the expanded state, the valve stent transitions from the axial middle portion toward the first axial end sequentially through a first inwardly concave arcuate segment and a first outwardly convex arcuate segment, and transitions from the axial middle portion toward the second axial end sequentially through a second inwardly concave arcuate segment and a second outwardly convex arcuate segment, where a cross section of the first inwardly concave arcuate segment passing through an axis of the valve stent is a first inwardly concave arc having a radius R_{A} and recessed toward an inner side of the valve stent; a cross section of the first outwardly convex arcuate segment passing through the axis of the valve stent is a first outwardly convex arc having a radius R_{B} and protruding toward an outer side of the valve stent; a cross section of the second inwardly concave arcuate segment passing through the axis of the valve stent is a second inwardly concave arc having a radius R_{C} and recessed toward the inner side of the valve stent; and a cross section of the second outwardly convex arcuate segment passing through the axis of the valve stent is a second outwardly convex arc having a radius R_{D} and protruding toward the outer side of the valve stent; where
R_{A} and R_{C} each range from 3.0 mm to 15.0 mm, and R_{B} and R_{D} each range from 3.0 mm to 15.0 mm.

According to a second aspect, the present disclosure provides a method for forming the valve stent according to the first aspect of the present disclosure. The processing method includes:
a stent cutting step, including: cutting metal tubing to form a blank having the same structure as the valve stent in a post-cutting state, and causing the two stent struts that are arranged opposite each other in the circumferential direction of the valve stent in each quadrilateral grid structure of the blank to gradually diverge from each other from the axial middle portion toward the axial end portion of the quadrilateral grid structure to form an included angle, where the included angle of the middle supporting grid structure formed by the straight segments of the two stent struts that are arranged opposite each other in the circumferential direction of the valve stent is less than or equal to 12°;
a stent pre-shaping step, including: performing multi-step pre-shaping on the blank in the post-cutting state and performing annealing after each pre-shaping step to form a blank in an expanded state; and
a stent shaping step, including: shaping the blank in the expanded state after the stent pre-shaping step by using a shaping mold to form the valve stent in the expanded state.

In the processing method according to some embodiments, in the stent pre-shaping step, the multi-step pre-shaping includes 2 to 6 pre-shaping steps; and/or
a maximum strain in each pre-shaping step is less than 12%; and/or
the annealing treatment is performed at an annealing temperature of 400 °C to 500 °C, with a holding time of 2 min to 21 min, and a cooling mode of water cooling.

In the processing method according to some embodiments, the stent shaping step includes:
fixing the blank in the expanded state within the shaping mold; and
performing annealing shaping treatment on the blank fixed within the shaping mold, where the annealing shaping treatment is performed at an annealing temperature of 500 °C to 550 °C, with a holding time of 2 min to 30 min, and a cooling mode of water cooling.

According to a third aspect, the present disclosure further provides a artificial heart valve including the valve stent, where the valve stent is the valve stent according to the first aspect of the present disclosure.

Based on the valve stent, the method for forming the valve stent, and the artificial heart valve provided by the present disclosure, when the valve stent is in the post-cutting state, the straight segments of the two stent struts that are arranged opposite each other in the circumferential direction of the valve stent in each quadrilateral grid structure gradually diverge from each other from the axial middle portion toward the axial end portion of the quadrilateral grid structure to form an included angle, where the included angle of the middle supporting grid structure formed by the straight segments of the two stent struts that are arranged opposite each other in the circumferential direction of the valve stent is less than or equal to 12°, thereby facilitating the alleviation or resolution of the problem that the valve stent formed by cutting a metal tubing is prone to fracture during processing, and further improving safety and clinical efficacy during use.

Other features and advantages of the present disclosure will become apparent from the following detailed description of exemplary embodiments of the present disclosure with reference to the accompanying drawings.

### BRIEF DESCRIPTION OF THE DRAWINGS

The accompanying drawings described herein are provided for further understanding of the present disclosure and constitute a part of the present disclosure. The exemplary embodiments of the present disclosure and the descriptions thereof are provided for explaining the present disclosure and do not constitute an improper limitation of the present disclosure.
FIG. 1 is a schematic diagram of a three-dimensional structure of a artificial heart valve according to an embodiment of the present disclosure viewed from one direction, where a valve stent is in an expanded state.
FIG. 2 is a schematic diagram of a three-dimensional structure of the artificial heart valve according to the embodiment of the present disclosure viewed from another direction, where the valve stent is in the expanded state.
FIG. 3 is a schematic diagram of a three-dimensional structure of the artificial heart valve according to the embodiment of the present disclosure viewed from still another direction, where the valve stent is in the expanded state, and a sealing membrane is removed.
FIG. 4 is a schematic diagram of a three-dimensional structure of a valve stent according to an embodiment of the present disclosure in a post-cutting state.
FIG. 5 is a schematic diagram of a front-view structure of a valve stent according to an embodiment of the present disclosure in a post-cutting state.
FIGS. 6-9 are schematic diagrams of a structure of one grid structure unit of the valve stent in the post-cutting state shown in FIG. 5.
FIG. 10 is a schematic diagram of a front-view structure of a valve stent according to an embodiment of the present disclosure in an expanded state.
FIG. 11 is a schematic diagram of a structure of a valve stent according to an embodiment of the present disclosure in an expanded state, where a sealing membrane is removed, and a finite element mesh used for finite element analysis is shown.
FIG. 12 is a flowchart of a method for forming a valve stent according to an embodiment of the present disclosure.

### DETAILED DESCRIPTION

The following clearly and completely describes the technical solutions in the embodiments of the present disclosure with reference to the accompanying drawings in the embodiments of the present disclosure. Apparently, the described embodiments are merely a part rather than all of the embodiments of the present disclosure. The following description of at least one exemplary embodiment is merely illustrative in nature and is in no way intended to limit the present disclosure or any application or use of the present disclosure. Based on the embodiments of the present disclosure, all other embodiments that can be obtained by those having ordinary skill in the art without inventive efforts shall fall within the protection scope of the present disclosure.

Unless otherwise specifically indicated, the relative arrangement of components and steps, numerical expressions, and numerical values set forth in these embodiments do not limit the scope of the present disclosure. Meanwhile, it should be understood that, for ease of description, the dimensions of various portions shown in the accompanying drawings are not necessarily drawn to scale. The technologies, methods, and devices known to those of ordinary skill in the art may not be discussed in detail; however, where appropriate, such technologies, methods, and devices shall be regarded as forming part of the specification. In all examples illustrated and discussed herein, any specific value shall be construed merely as an example rather than as a limitation. Therefore, other examples of the exemplary embodiments may have different values. It should be noted that similar reference numerals and letters indicate similar items in the following drawings, and therefore, once an item is defined in one of the drawings, no further discussion is required in the following drawings.

In the description of the present disclosure, it should be understood that terms such as "first" and "second" are used merely for distinguishing corresponding components. Unless otherwise expressly stated, such terms do not have any special meaning and therefore shall not be construed as limiting the protection scope of the present disclosure.

In the description of the present disclosure, it should be understood that orientation terms such as "front", "rear", "upper", "lower", "left", "right", "transverse", "longitudinal", "vertical", "horizontal", "top", and "bottom" generally indicate orientations or positional relationships shown in the accompanying drawings and are used only for convenience in describing the present disclosure and simplifying the description. Unless otherwise expressly stated, such orientation terms neither indicate nor imply that the referenced device or component must have a particular orientation or be constructed and operated in a particular orientation, and therefore shall not be construed as limiting the protection scope of the present disclosure. In addition, the orientation terms "inner" and "outer" refer to the inside and the outside relative to the contour of each component.

Furthermore, the technical features involved in different embodiments of the present disclosure described below may be combined with each other provided that no conflict exists therebetween.

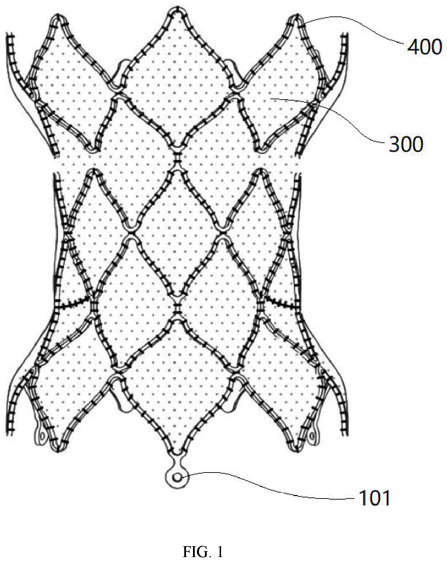

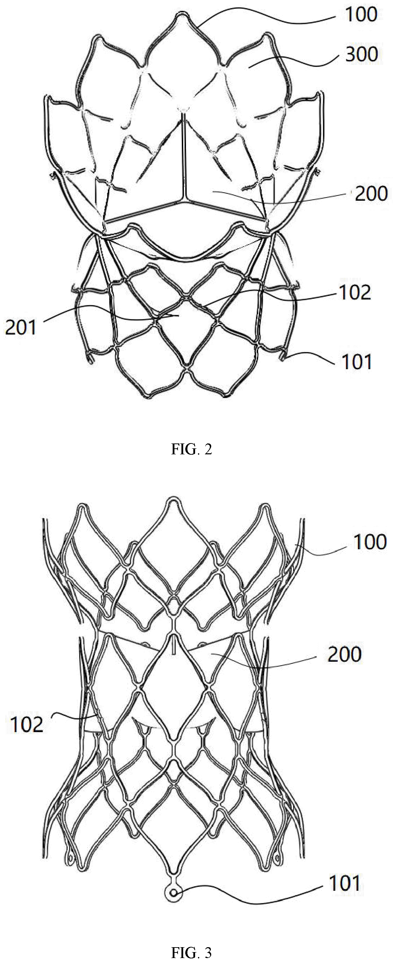

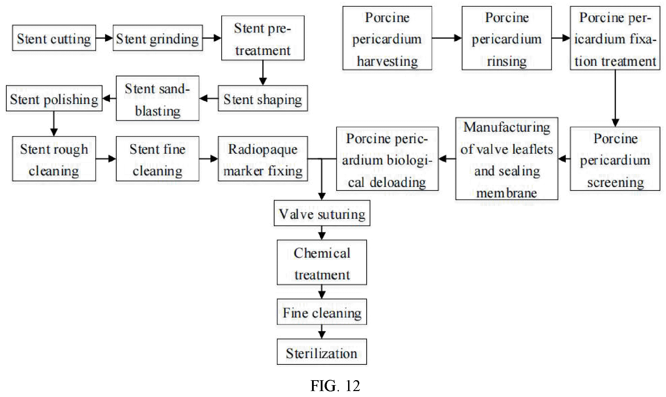

As shown in FIGS. 1 to 11, an embodiment of the present disclosure provides a artificial heart valve and a valve stent 100 of the artificial heart valve. As shown in FIG. 12, an embodiment of the present disclosure further provides a method for forming the valve stent 100 and the artificial heart valve.

As shown in FIGS. 1 to 11, the valve stent 100 according to the embodiment of the present disclosure is formed by cutting metal tubing and has a post-cutting state and an expanded state. The post-cutting state refers to an initial geometric configuration in which a blank of the valve stent 100 has just been cut and formed from the metal tubing but has not yet undergone any subsequent processing procedures, including expansion, shape setting, or heat treatment. The expanded state refers to a working geometric configuration in which the blank of the valve stent 100 has reached, after undergoing a series of processing procedures including, but not limited to, multi-step pre-shaping, annealing, and final shape setting, for providing a supporting function after implantation into a body. In other words, the "post-cutting state" is a semi-finished configuration of the valve stent 100 during the manufacturing process, and the "expanded state" is a final product configuration intended to be achieved.

The valve stent 100 includes a plurality of grid structure units A distributed along a circumferential direction of the valve stent 100, and the grid structure units A extend along an axial direction of the valve stent 100. Each grid structure unit A includes a plurality of quadrilateral grid structures. The plurality of quadrilateral grid structures include at least one near-end grid structure 120, at least one middle supporting grid structure 130, and at least one far-end grid structure 140, which are sequentially arranged along the axial direction of the valve stent 100 from a first axial end to a second axial end. Each quadrilateral grid includes four stent struts B connected end to end in sequence. The stent strut B includes a straight segment B₁ and a connecting segment B₂. The straight segment B₁ of each stent strut B forms a grid body of the quadrilateral grid structure. The connecting segment B₂ of each stent strut B forms a corner portion of a corresponding quadrilateral grid structure, adjacent stent struts B of each quadrilateral grid structure are connected through the connecting segments B₂, and corner portions at axial middle portions of corresponding quadrilateral grid structures of adjacent grid structure units A are connected.

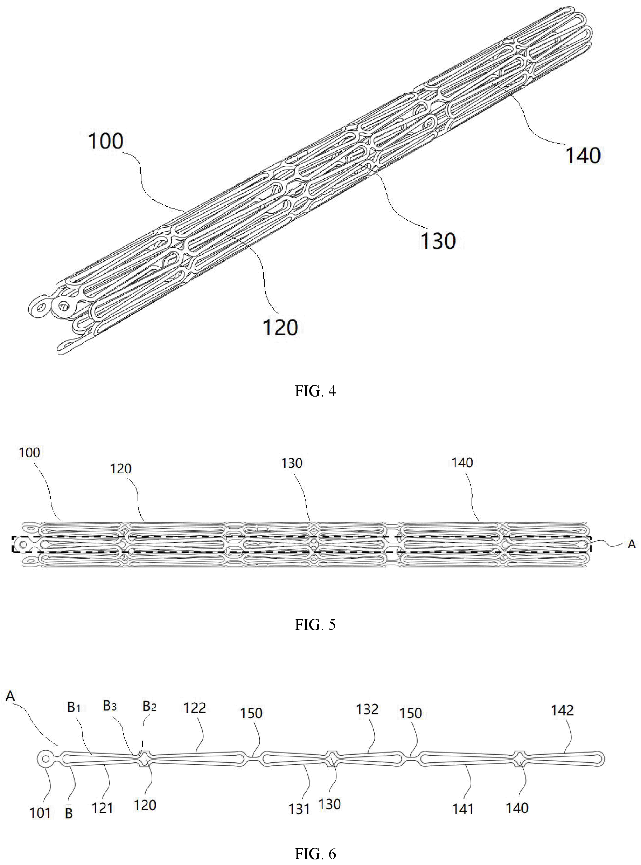

As shown in FIGS. 4 to 9, when the valve stent 100 is in the post-cutting state, the straight segments B₁ of the two stent struts B that are arranged opposite each other in the circumferential direction of the valve stent 100 in each quadrilateral grid structure gradually diverge from each other from the axial middle portion toward the axial end portion of the quadrilateral grid structure to form an included angle, where the included angle of the middle supporting grid structure 130 formed by the straight segments B₁ of the two stent struts B that are arranged opposite each other in the circumferential direction of the valve stent 100 is less than or equal to 12°.

According to the valve stent 100 in the embodiment of the present disclosure, when the valve stent 100 is in the post-cutting state, the straight segments B₁ of the two stent struts B that are arranged opposite each other in the circumferential direction of the valve stent 100 in each quadrilateral grid structure gradually diverge from each other from the axial middle portion toward the axial end portion of the quadrilateral grid structure to form an included angle, where the included angle of the middle supporting grid structure 130 formed by the straight segments B₁ of the two stent struts B that are arranged opposite each other in the circumferential direction of the valve stent 100 is less than or equal to 12°, thereby facilitating the alleviation or resolution of the problem that the valve stent formed by cutting a metal tubing is prone to fracture during processing of the valve stent 100, and further improving safety and clinical efficacy during use.

In FIGS. 5 to 9, the axial direction of the valve stent 100 and the grid structure unit A corresponds to a left-right direction in the figures, the first axial end corresponds to a left side in the figures, and the second axial end corresponds to a right side in the figures.

In the valve stent according to some embodiments, the plurality of quadrilateral grid structures include two middle supporting grid structures 130, and an included angle of each middle supporting grid structure 130 formed by the straight segments B₁ of the two stent struts B that are arranged opposite each other in the circumferential direction of the valve stent 100 in is less than 11.74°.

The included angle in two layers of the middle supporting grid structures 130 formed by the straight segments B₁ of the two stent struts B that are arranged opposite each other in the circumferential direction of the valve stent 100 (hereinafter also referred to as a straight segment cutting angle) is less than 11.74°, thereby further facilitating alleviation or resolution of the problem that the valve stent 100 formed by cutting a metal tubing is prone to fracture during processing, and further improving safety and clinical efficacy during use.

In the valve stent according to some embodiments, the plurality of quadrilateral grid structures include two middle supporting grid structures 130, and the included angle of each middle supporting grid structure 130 formed by the straight segments B₁ of the two stent struts B that are arranged opposite each other in the circumferential direction of the valve stent 100 is 5.0° to 11.0°.

The straight segment cutting angle of the two layers of the middle supporting grid structures 130 in a range of 5.0° to 11.0° enables more optimal comprehensive performance of the valve stent 100 in aspects of processing, loading and releasing, and fatigue failure resistance.

In the valve stent according to some embodiments, the plurality of quadrilateral grid structures include one middle supporting grid structure 130, and an included angle of the middle supporting grid structure 130 formed by the straight segments B₁ of the two stent struts B that are arranged opposite each other in the circumferential direction of the valve stent 100 is less than 6.14°.

A straight segment cutting angle of one layer of the middle supporting grid structure 130 that is less than 6.14° enables favorable comprehensive performance of the valve stent 100 in aspects of processing, loading and releasing, and fatigue failure resistance.

In the valve stent according to some embodiments, the plurality of quadrilateral grid structures include one middle supporting grid structure 130, and the included angle of the middle supporting grid structure 130 formed by the straight segments B₁ of the two stent struts B that are arranged opposite each other in the circumferential direction of the valve stent 100 is 2.5° to 5.5°.

The straight segment cutting angle of the one layer of the middle supporting grid structure 130 in a range of 2.5° to 5.5° enables more optimal comprehensive performance of the valve stent 100 in aspects of processing, loading and releasing, and fatigue failure resistance.

For example, in the valve stent 100 according to some embodiments, in view of the shaping, crimping, and fatigue performance in a subsequent working state of the stent, the plurality of quadrilateral grid structures include one middle supporting grid structure 130. When the valve stent 100 is in the post-cutting state, the included angle of the middle supporting grid structure 130 formed by the straight segments B₁ of the two stent struts B that are arranged opposite each other in the circumferential direction of the valve stent 100 is, for example, 2.83° to 4.67°.

The straight segment cutting angle of the middle supporting grid structure 130 in a range of 2.83° to 4.67° enables optimal comprehensive performance of the valve stent 100 in aspects of processing, loading and releasing, and fatigue failure avoidance.

In the valve stent 100 according to some embodiments, the plurality of quadrilateral grid structures include one near-end grid structure 120, at least one middle supporting grid structure 130, and one far-end grid structure 140, which are sequentially arranged along the axial direction of the valve stent 100 from the first axial end to the second axial end. In an embodiment not shown, more than one near-end grid structures 120, more than one middle supporting grid structures 130, and more than one far-end grid structures 140 may be provided.

In the valve stent 100 according to some embodiments, when the valve stent 100 is in the post-cutting state, an included angle of the middle supporting grid structure 130 formed by the straight segments B₁ of the two stent struts B adjacent to the first axial end is equal to or different from an included angle of the middle supporting grid structure 130 formed by the straight segments B₁ of the two stent struts B adjacent to the second axial end.

In this configuration, the deformation of the middle supporting grid structure 130 is more uniform and consistent, thereby facilitating alleviation of stress concentration.

In an embodiment not shown, the included angle of the middle supporting grid structure 130 formed by the straight segments B₁ of the two stent struts B adjacent to the first axial end may also be not equal to the included angle formed by the straight segments B₁ of the two stent struts B adjacent to the second axial end.

As shown in FIGS. 4 to 9, in the valve stent 100 according to some embodiments, the connecting segment B₂ of the stent strut B includes two curved connecting segments respectively disposed at two ends of the straight segment B₁, and adjacent stent struts B of each quadrilateral grid structure are connected through the curved connecting segment; a curved connecting segment of the stent strut B adjacent to an axial end portion of the quadrilateral grid structure in which the stent strut B is located is curved toward an inner side of the quadrilateral grid structure; and a curved connecting segment of the stent strut B adjacent to an axial middle portion of the quadrilateral grid structure in which the stent strut B is located is curved toward an outer side of the quadrilateral grid structure.

The provision of the curved connecting segments and a bending direction of the curved connecting segments facilitates the reduction of stress concentration, thereby facilitating the reduction of fracture during shaping processing, and also enabling easier crimping deformation during use.

As shown in FIGS. 4 to 9, in the valve stent 100 according to some embodiments, the curved connecting segment is an arcuate connecting segment, and a ratio of a length of the straight segment B₁ to an inner radius R₁ of the arcuate connecting segment is in a range of 4 to 30. For example, the ratio of the length of the straight segment B₁ to the inner radius of the arcuate connecting segment may be 13, the inner radius R₁ of the arcuate connecting segment may be 0.5 mm, and the length of the straight segment B₁ may be 7.5 mm.

The ratio of the inner radius of the arcuate connecting segment and the length of the straight segment may be determined based on a number of grid structure units A, a cutting space of the stent, and a width of the stent strut B. The number of grid structure units A may be, for example, 6, 9, or 12. A larger value under a feasible condition enables better performance of the valve stent in shaping, crimping, and fatigue performance in a subsequent working state.

The inner radius R₁ of the arcuate connecting segment may range from 0.25 mm to 0.93 mm. For example, in an embodiment, the number of grid structure units A is 9, and the inner radius R₁ of the corresponding arcuate connecting segment may be 0.49 mm.

As shown in FIGS. 4 to 9, in the valve stent 100 according to some embodiments, the straight segment B₁ is connected to the curved connecting segment through a curved transition segment B₃, and a bending direction of the curved transition segment B₃ is opposite to a bending direction of the curved connecting segment.

An Ω-shaped structure in which the bending direction of the curved transition segment B₃ is opposite to the bending direction of the curved connecting segment adopted by the stent strut B enables stress concentration to be distributed over an entire arcuate connecting segment, thereby facilitating the prevention of fracture during shaping processing, and also enabling easier crimping deformation during use.

As shown in FIGS. 4 to 9, in the valve stent 100 according to some embodiments, the curved transition segment B₃ is an arcuate transition segment, and a ratio of an inner radius of the arcuate transition segment to the length of the straight segment B₁ is in a range of 0.25 to 0.75.

The ratio of the inner radius of the arcuate transition segment to the length of the straight segment B₁ in the range of 0.25 to 0.75 facilitates more effective alleviation of stress concentration and reduction of fracture during shaping processing, and also enables easier crimping deformation during use. For example, in a preferred embodiment, the ratio of the inner radius of the arcuate transition segment to the length of the straight segment B₁ may be 0.48.

The inner radius of the arcuate transition segment may be in a range of 2.11 mm to 4.55 mm. The stress concentration at the connection between the straight segment and the arcuate segment is only at a single tangent point. This Ω-shaped structure enables stress concentration to be distributed over an entire arcuate connecting segment, thereby preventing fracture during shaping processing, and also enabling easier crimping deformation during use. The range of the inner radius enables effective utilization of the Ω-shaped structure to alleviate stress concentration.

In the valve stent 100 according to some embodiments, the curved connecting segment is an arcuate connecting segment. A length of the near-end grid structure 120 along the axial direction of the valve stent 100 between the centers of the arcuate connecting segments at two ends of the stent strut B adjacent to the first axial end may be equal to or different from a length of the near-end grid structure 120 along the axial direction of the valve stent 100 between the centers of the arcuate connecting segments at two ends of the stent strut B adjacent to the second axial end; and/or
a length of the far-end grid structure 140 along the axial direction of the valve stent 100 between the centers of the arcuate connecting segments at two ends of the stent strut B adjacent to the first axial end may be equal to or different from a length of the far-end grid structure 140 along the axial direction of the valve stent 100 between the centers of the arcuate connecting segments at two ends of the stent strut B adjacent to the second axial end; and/or
a length of the middle supporting grid structure 130 along the axial direction of the valve stent 100 between the centers of the arcuate connecting segments at two ends of the stent strut B adjacent to the first axial end is equal to or different from a length of the middle supporting grid structure 130 along the axial direction of the valve stent 100 between the centers of the arcuate connecting segments at two ends of the stent strut B adjacent to the second axial end.

For example, in the middle supporting grid structure 130, a configuration in which the stent strut B at the first axial end and the second axial end are of equal length facilitates more uniform and consistent deformation of the quadrilateral grid structure, thereby facilitating the alleviation of stress concentration. In the quadrilateral far-end grid structure and the quadrilateral near-end grid structure, a configuration in which the stent strut B at the first axial end and the second axial end are of unequal length facilitates the deformation of the quadrilateral grid structure, such that the valve stent 100 forms a non-equal-diameter shape in the expanded state.

In the valve stent 100 according to some embodiments, the length of the near-end grid structure 120 along the axial direction of the valve stent 100 between the centers of the arcuate connecting segments at two ends of the stent strut B adjacent to the first axial end is less than the length of the near-end grid structure 120 along the axial direction of the valve stent 100 between the centers of the arcuate connecting segments at two ends of the stent strut B adjacent to the second axial end; and/or
the length of the far-end grid structure 140 along the axial direction of the valve stent 100 between the centers of the arcuate connecting segments at two ends of the stent strut B adjacent to the first axial end is greater than the length of the far-end grid structure 140 along the axial direction of the valve stent 100 between the centers of the arcuate connecting segments at two ends of the stent strut B adjacent to the second axial end.

A length of the stent strut B at the first axial end of the near-end grid structure 120 is less than a length at the second axial end of the near-end grid structure 120, which facilitates the deformation of each near-end grid structure 120 such that, in the expanded state, a diameter of a portion of the valve stent 100 located at the near-end grid structure 120 at the first axial end is greater than a diameter at the second axial end.

A length of the stent strut B at the first axial end of the far-end grid structure 140 is greater than a length at the second axial end of the far-end grid structure 140, which facilitates the deformation of each far-end grid structure 140 such that, in the expanded state, a diameter of a portion of the valve stent 100 located at the far-end grid structure 140 at the second axial end is greater than a diameter at the first axial end.

In the valve stent 100 according to some embodiments, a ratio of the length of the near-end grid structure 120 along the axial direction of the valve stent 100 between the centers of the arcuate connecting segments at two ends of the stent strut B adjacent to the first axial end and the length of the near-end grid structure 120 along the axial direction of the valve stent 100 between the centers of the arcuate connecting segments at two ends of the stent strut B adjacent to the second axial end is 0.65 to 1.00; and/or
a ratio of the length of the far-end grid structure 140 along the axial direction of the valve stent 100 between the centers of the arcuate connecting segments at two ends of the stent strut B adjacent to the second axial end and the length of the far-end grid structure 140 along the axial direction of the valve stent 100 between the centers of the arcuate connecting segments at two ends of the stent strut B adjacent to the first axial end is 0.65 to 1.00.

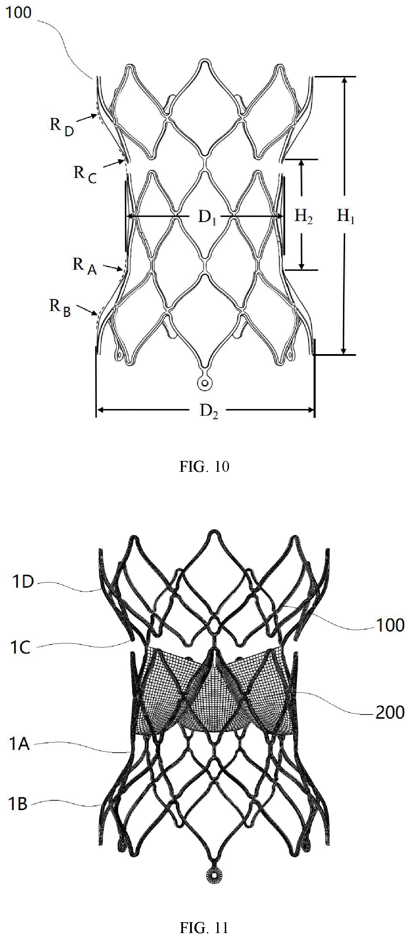

As shown in FIGS. 10 and 11, the ratio of the length of the near-end grid structure 120 along the axial direction of the valve stent 100 between the centers of the arcuate connecting segments at two ends of the stent strut B adjacent to the first axial end and the length of the near-end grid structure 120 along the axial direction of the valve stent 100 between the centers of the arcuate connecting segments at two ends of the stent strut B adjacent to the second axial end may be determined based on a position of a center point of a first outwardly convex arc (the arc having a radius labeled R_{B}) of a first outwardly convex arcuate segment 1B of the valve stent 100 in the expanded state, the length of the stent strut B adjacent to the second axial end of the near-end grid structure 120 is appropriately increased, and the length of the stent strut B adjacent to the first axial end of the near-end grid structure 120 is appropriately reduced in order to control an overall length of the valve stent 100, thereby facilitating the reduction of fracture risk during shaping.

Similarly, as shown in FIGS. 10 and 11, the ratio of the length of the far-end grid structure 140 along the axial direction of the valve stent 100 between the centers of the arcuate connecting segments at two ends of the stent strut B adjacent to the second axial end and the length of the far-end grid structure 140 along the axial direction of the valve stent 100 between the centers of the arcuate connecting segments at two ends of the stent strut B adjacent to the first axial end may be determined based on a position of a center point of a second outwardly convex arc (the arc having a radius labeled R_{D}) of a second outwardly convex arcuate segment 1D of the valve stent 100 in the expanded state, the length of the stent strut B adjacent to the first axial end of the far-end grid structure 140 is appropriately increased, and the length of the stent strut B adjacent to the second axial end of the far-end grid structure 140 is appropriately reduced in order to control an overall length of the valve stent 100, thereby facilitating the reduction of fracture risk during shaping.

In the valve stent 100 according to some embodiments, the curved connecting segment is an arcuate connecting segment; and the plurality of quadrilateral grid structures include one near-end grid structure 120, at least one middle supporting grid structure 130, and one far-end grid structure 140, which are sequentially arranged along the axial direction of the valve stent 100 from the first axial end to the second axial end. A length of the middle supporting grid structure 130 along the axial direction of the valve stent 100 between centers of arcuate connecting segments at two ends of each stent strut B is in a range of 3.05 mm to 8.22 mm. For example, in some embodiments, the length of the middle supporting grid structure 130 along the axial direction of the valve stent 100 between the centers of the arcuate connecting segments at two ends of each stent strut B may be 6.45 mm.

A length of the near-end grid structure 120 along the axial direction of the valve stent 100 between the centers of the arcuate connecting segments at two ends of each stent strut B is in a range of 3.55 mm to 12.50 mm. For example, a length of the near-end grid structure 120 along the axial direction of the valve stent 100 between centers of arcuate connecting segments at two ends of each stent strut B adjacent to the first axial end is 7.93 mm; and a length of the near-end grid structure 120 along the axial direction of the valve stent 100 between centers of arcuate connecting segments at two ends of each stent strut B adjacent to the second axial end is 9.91 mm.

A length of the far-end grid structure 140 along the axial direction of the valve stent 100 between the centers of the arcuate connecting segments at two ends of each stent strut B is in a range of 3.55 mm to 12.50 mm. For example, a length of the far-end grid structure 140 along the axial direction of the valve stent 100 between centers of arcuate connecting segments at two ends of each stent strut B adjacent to the first axial end is 9.91 mm; and a length of the far-end grid structure 140 along the axial direction of the valve stent 100 between centers of arcuate connecting segments at two ends of each stent strut B adjacent to the second axial end is 7.93 mm.

In the valve stent 100 according to some embodiments, the curved connecting segment is an arcuate connecting segment; and for each grid structure unit,
${L}_{i} \times tan \frac{{α}_{i}}{2} = {L}_{j} \times tan \frac{{α}_{j}}{2} .$

Lᵢ is a length of the quadrilateral grid structure along the axial direction of the valve stent 100 between the centers of the arcuate connecting segments at two ends of the stent strut B adjacent to the first axial end; Lⱼ is a length of the quadrilateral grid structure along the axial direction of the valve stent 100 between the centers of the arcuate connecting segments at two ends of the stent strut B adjacent to the second axial end; αᵢ is an included angle of the quadrilateral grid structure formed by straight segments B₁ of two stent struts B adjacent to the first axial end; and αⱼ is an included angle of the quadrilateral grid structure formed by straight segments B₁ of two stent struts B adjacent to the second axial end.

Only αᵢ and αⱼ of the middle supporting grid structure 130 need to be controlled, and the lengths Lᵢ and Lⱼ of different supporting grid structures need to be adjusted, so that the dimensions of an entire valve stent in the post-cutting state can be obtained, thereby facilitating parameter optimization of an overall valve stent structure.

In the valve stent 100 according to some embodiments, the valve stent 100 further includes a plurality of connecting rods 150, where
the near-end grid structure 120 and the middle supporting grid structure 130 of each grid structure unit A are connected through one connecting rod 150; or, along the circumferential direction of the valve stent 100, one grid structure unit A in which the near-end grid structure 120 and the middle supporting grid structure 130 are connected through one connecting rod 150 is provided for every one or two grid structure units A in which the near-end grid structure 120 is not connected to the middle supporting grid structure 130 through the connecting rod 150; and/or
the far-end grid structure 140 and the middle supporting grid structure 130 of each grid structure unit A are connected through one connecting rod 150; or, along the circumferential direction of the valve stent 100, one grid structure unit A in which the far-end grid structure 120 and the middle supporting grid structure 130 are connected through one connecting rod 150 is provided for every one or two grid structure units A in which the far-end grid structure 140 is not connected to the middle supporting grid structure 130 through the connecting rod 150.

At the same axial position of the valve stent 100, a greater number of connecting rods 150 in the circumferential direction enables the valve stent 100 to provide greater supporting force at the corresponding position, and a smaller number of connecting rods 150 in the circumferential direction enables the valve stent 100 to have better flexibility and compliance at the corresponding position.

For example, in a patient having a pulmonary artery lesion, a distal end has a more complex shape than a proximal end and is more likely to have a non-circular profile, and the proximal end and a valve annulus require greater radial supporting force to maintain a physiological circular shape of the pulmonary artery after implantation of the artificial heart valve. The near-end grid structures 120 and the middle supporting grid structures 130 of all grid structure units A of the valve stent 100 may be connected through the connecting rods 150, such that a proximal end of the valve stent 100 forms a closed-loop structure. The near-end grid structures 120 and the middle supporting grid structures 130 of a first portion of the grid structure units A of the valve stent 100 may be connected through the connecting rods 150, the near-end grid structures 120 and the middle supporting grid structures 130 of a remaining second portion of the grid structure units A are not connected, and the first portion of the grid structure units A and the second portion of the grid structure units A are alternately arranged in a predetermined pattern, such that a distal end of the valve stent 100 forms a semi-open-loop structure. After implantation of the artificial heart valve, the valve stent 100 provides relatively high overall radial supporting force at the proximal end, thereby facilitating the maintenance of a physiological circular shape of the proximal end. The valve stent 100 has favorable flexibility and compliance at the distal end, thereby facilitating favorable wall apposition of the artificial heart valve. Therefore, the risk of paravalvular leakage at two ends of the artificial heart valve can be reduced, and the overall fatigue resistance of the valve stent 100 under torsional movement of a right heart system is improved.

In the valve stent 100 according to some embodiments, the connecting rod 150 of the stent strut B is a straight rod, an S-shaped rod, an M-shaped rod, or an N-shaped rod. The shapes of different connecting rods 150 affect an axial shortening ratio of the valve stent 100 in the expanded state, which has different effects on accurate positioning of the valve stent 100.

As shown in FIG. 10, in the valve stent 100 according to some embodiments, when the valve stent 100 is in the expanded state, both an outer diameter D₂ at the first axial end and an outer diameter D₂ at the second axial end of the valve stent 100 are greater than an outer diameter D₁ at the axial middle portion.

The valve stent 100 forms a dumbbell-shaped structure in the expanded state. Vascular morphologies of patients with right ventricular pulmonary artery are complex and variable, and enlargement deformation occurs in most cases. The valve stent 100 has a dumbbell-shaped structure in the expanded state, which facilitates the prevention of upward and downward displacement of the artificial heart valve and is more suitable for patients having a native outflow tract.

In the valve stent 100 according to some embodiments, in the expanded state, the outer diameter D₁ at the axial middle portion of the valve stent 100 is in a range of 18.0 mm to 32.0 mm; and in the expanded state, the outer diameter D₂ at the first axial end and the outer diameter D₂ at the second axial end of the valve stent 100 is in a range of 20.0 mm to 54.0 mm. D₁ may be, for example, 20 mm, 23 mm, 26 mm, or 29 mm, and D₂ may be, for example, 28 mm, 32 mm, 36 mm, 40 mm, 44 mm, 48 mm, or 52 mm.

The reasonable setting of the range of D₁ enables the dimensional range of D₁ to match, to the greatest extent possible, an original size of a valve root of a patient, thereby making hemodynamics more stable. The reasonable setting of the range of D₂ facilitates better fixation and fitting of the valve stent according to the morphology of a patient's pulmonary artery after lesion, and prevents the occurrence of paravalvular leakage.

In the valve stent according to some embodiments, in the expanded state, a total height H₁ of the valve stent 100 is in a range of 30.0 mm to 60.0 mm, and a height H₂ of the middle supporting grid structure 130 is in a range of 12.0 mm to 30.0 mm. H₁ may be, for example, 36 mm, 38 mm, 42 mm, 46 mm, 50 mm, 52 mm, or 54 mm. H₂ may be, for example, 14 mm, 15 mm, 16 mm, 18 mm, 20 mm, or 24 mm.

The reasonable setting of the ranges of H₁ and H₂ enables the dimension of H₁ to reduce an influence on pulmonary artery tissue after implantation while satisfying processing conditions, and enables the dimensional range of H₂ to vary according to a height of sutured valve leaflets.

As shown in FIGS. 10 and 11, in the valve stent 100 according to some embodiments, when the valve stent 100 is in the expanded state, the valve stent 100 transitions from the axial middle portion toward the first axial end sequentially through a first inwardly concave arcuate segment 1A and a first outwardly convex arcuate segment 1B, and transitions from the axial middle portion toward the second axial end sequentially through a second inwardly concave arcuate segment 1C and a second outwardly convex arcuate segment 1D;
a cross section of the first inwardly concave arcuate segment 1A passing through an axis of the valve stent 100 is a first inwardly concave arc having a radius R_{A} and recessed toward an inner side of the valve stent 100; a cross section of the first outwardly convex arcuate segment 1B passing through the axis of the valve stent 100 is a first outwardly convex arc having a radius R_{B} and protruding toward an outer side of the valve stent 100; a cross section of the second inwardly concave arcuate segment 1C passing through the axis of the valve stent 100 is a second inwardly concave arc having a radius R_{C} and recessed toward the inner side of the valve stent 100; and a cross section of the second outwardly convex arcuate segment 1D passing through the axis of the valve stent 100 is a second outwardly convex arc having a radius R_{D} and protruding toward the outer side of the valve stent 100.

The provision of the first inwardly concave arcuate segment 1A, the first outwardly convex arcuate segment 1B, the second inwardly concave arcuate segment 1C, and the second outwardly convex arcuate segment 1D enables all arcuate segments to serve as transition segments during a diameter variation process of the valve stent 100. The smooth transition facilitates the prevention of hemodynamic disorder caused by an abrupt change in a cross-sectional shape or size, thereby reducing risks of unexpected thrombosis and the like.

In the valve stent 100 according to some embodiments, R_{A} and R_{C} each range from 3.0 mm to 15.0 mm, and R_{B} and R_{D} each range from 3.0 mm to 15.0 mm. R_{D} and R_{B} may be equal, for example, 5 mm, 6 mm, 7 mm, 8 mm, 9 mm, or 10 mm. R_{C} and R_{A} may be equal, for example, 5 mm, 7 mm, 9 mm, 10 mm, 11 mm, or 13 mm.

The reasonable setting of the ranges of R_{D} and R_{B} as well as R_{C} and R_{A} facilitates the reduction of the risk of fracture during shaping and crimping of the valve stent, and also ensures a longer fatigue service life after implantation into a human body.

As shown in FIG. 12, the method for forming the valve stent according to the embodiment of the present disclosure includes the following steps.
a stent cutting step, including: cutting metal tubing to form a blank having the same structure as the valve stent 100 in a post-cutting state, and causing the two stent struts B that are arranged opposite each other in the circumferential direction of the valve stent 100 in each quadrilateral grid structure of the blank to gradually diverge from each other from the axial middle portion toward the axial end portion of the quadrilateral grid structure to form an included angle, where the included angle of the middle supporting grid structure 130 formed by the straight segments B₁ of the two stent struts B that are arranged opposite each other in the circumferential direction of the valve stent 100 is less than or equal to 12°;
a stent pre-shaping step, including: performing multi-step pre-shaping on the blank in the post-cutting state and performing annealing after each pre-shaping step to form a blank in an expanded state; and
a stent shaping step, including: shaping the blank in the expanded state after the stent pre-shaping step by using a shaping mold to form the valve stent 100 in the expanded state.

By using the above processing method, the valve stent 100 according to the embodiments of the present disclosure can be implemented. Therefore, the valve stent 100 manufactured by the processing method according to the embodiment of the present disclosure has the advantages of the valve stent 100 according to the embodiment of the present disclosure.

In the processing method according to some embodiments, the stent pre-shaping step includes performing multi-step pre-shaping on a blank stent, and performing annealing treatment after each pre-shaping step.

The multi-step pre-shaping and the annealing treatment after each pre-shaping step facilitate reduction of fracture or damage during the stent pre-shaping process by controlling a maximum strain in each step.

In the processing method according to some embodiments, in the stent pre-shaping step, the multi-step pre-shaping includes 2 to 6 pre-shaping steps; and/or the maximum strain in each pre-shaping step is less than 12%; and/or the annealing treatment is performed at an annealing temperature of 400 °C to 500 °C, with a holding time of 2 min to 21 min, and a cooling mode of water cooling.

The 2 to 6 pre-shaping steps and the maximum strain of less than 12% in each pre-shaping step facilitate maintaining deformation of the blank within an appropriate range during each pre-shaping step, thereby facilitating the reduction of fracture or damage during the stent pre-shaping process. The above annealing temperature and holding time facilitate effective removal of stress from the blank within a reasonable period of time, thereby facilitating subsequent processing.

In the processing method according to some embodiments, the stent shaping step includes: fixing the blank in the expanded state in a shaping mold; and performing annealing shaping treatment on the blank fixed in the shaping mold.

Shaping the blank by using the shaping mold facilitates obtaining a standardized valve stent 100, and the annealing shaping treatment facilitates the removal of stress from the valve stent 100 in the expanded state after completion of the stent shaping step.

In the processing method according to some embodiments, the annealing shaping treatment is performed at an annealing temperature of 500 °C to 550 °C, with a holding time of 2 min to 30 min, and a cooling mode of water cooling.

The above annealing temperature, holding time, and cooling mode facilitate effective removal of stress from the valve stent 100 in the expanded state within a reasonable period of time, and maintain the valve stent in a final shaped state without springback.

According to a third aspect, the present disclosure further provides a artificial heart valve including the valve stent 100, where the valve stent 100 is the valve stent 100 according to the first aspect of the present disclosure.

The artificial heart valve according to the embodiment of the present disclosure has the advantages of the valve stent 100 according to the embodiment of the present disclosure.

The artificial heart valve and the valve stent 100 of the artificial heart valve according to the embodiments of the present disclosure, as well as the method for forming the artificial heart valve and the valve stent of the artificial heart valve, are described in detail below with reference to FIGS. 1 to 12.

For convenience of description, the included angle formed by the straight segments B₁ of the two stent struts B that are arranged opposite each other in the circumferential direction of the valve stent 100 in each quadrilateral grid structure gradually diverging from the axial middle portion toward the axial end portion of the quadrilateral grid structure is also referred to as a straight segment cutting angle.

As shown in FIGS. 1 to 3, the artificial heart valve according to the embodiment of the present disclosure is a pulmonary valve. The artificial heart valve includes a valve stent 100, a valve 200, and a sealing membrane 300. The valve stent 100, the valve 200, and the sealing membrane 300 are sutured together by a suture 400. In the figures, 201 represents a valve edge. In the expanded state, diameters of the near-end grid structure 120 and the far-end grid structure 140 of the valve stent 100 gradually increase from the middle supporting grid structure 130 toward ends away from the middle supporting grid structure 130, thereby forming a dumbbell-shaped structure. A size of two stent struts B at the first axial end and a size of two stent struts B at the second axial end of the near-end grid structure 120 and the far-end grid structure 140 are different.

The artificial heart valve further includes a connecting claw 101 disposed on the valve stent 100. The connecting claw 101 is connected to a corner portion of the near-end grid structure 120 adjacent to the first axial end. The connecting claw 101 is configured to fix the artificial heart valve during implantation into a living body. A middle portion of the connecting claw 101 is provided with a connecting hole. The connecting hole may have a circular shape, a square shape, or a triangular shape. One connecting claw 101 may be provided for every two grid structure units A, or one connecting claw 101 may be provided for every one grid structure unit A, or one connecting claw 101 may be provided on each near-end grid structure 120 of each grid structure unit A.

In addition, the valve stent 100 is further provided with a fixing groove 102 for mounting a radiopaque material. The fixing groove 102 is disposed at the middle supporting grid structure 130 of the grid structure unit A.

As shown in FIGS. 4 to 9, the grid structure units A are symmetrically arranged with respect to a plane passing through the axis of the valve stent 100.

As shown in FIGS. 1 to 5 and FIG. 10, the near-end grid structure 120 and the middle supporting grid structure 130 of each grid structure unit A are connected through one connecting rod 150. The far-end grid structure 140 of each grid structure unit A is arranged such that, along the circumferential direction of the valve stent 100, one grid structure unit A in which the far-end grid structure 140 and the middle supporting grid structure 130 are connected through one connecting rod 150 is provided for every two grid structure units A in which the far-end grid structure 140 and the middle supporting grid structure 130 are not connected through the connecting rod 150. Therefore, a stiffness of the valve stent 100 at a connection structure between the near-end grid structure 120 and the middle supporting grid structure 130 is greater than a stiffness at a connection structure between the far-end grid structure 140 and the middle supporting grid structure 130, such that the valve stent 100 has a clearly asymmetric structure at two axial ends.

The valve stent 100 is formed by laser cutting a seamless metal tubing. The metal tubing may be a nickel-titanium alloy tubing. The nickel-titanium alloy tubing has good biocompatibility and mechanical properties, and can withstand blood flow and cardiac beating.

The nickel-titanium alloy tubing has an outer diameter in a range of 4.18 mm to 6.22 mm, a wall thickness in a range of 0.30 mm to 0.52 mm, and a length in a range of 88 mm to 2000 mm. A width W of a cut straight segment is generally in a range of 0.30 mm to 0.52 mm.

For example, in a specific embodiment, the nickel-titanium alloy tubing has the outer diameter of 4.2 mm, the wall thickness of 0.3 mm, and the length of 1500 mm, and the cut straight segment has the width W of 0.31 mm.

The stent strut B includes a straight segment B₁ and a connecting segment B₂. The connecting segment B₂ includes two arcuate connecting segments respectively disposed at two ends of the straight segment B₁, and adjacent stent struts B of the quadrilateral grid structure are connected through the arcuate connecting segments. An arcuate connecting segment of the stent strut B adjacent to an axial end portion of a quadrilateral grid structure in which the stent strut B is located is curved toward an inner side of the quadrilateral grid structure. An arcuate connecting segment of the stent strut B adjacent to an axial middle portion of the quadrilateral grid structure in which the stent strut B is located is curved toward an outer side of the quadrilateral grid structure. The straight segment B₁ is connected to the arcuate connecting segment through an arcuate transition segment. A bending direction of the arcuate transition segment is opposite to a bending direction of the arcuate connecting segment.

In the embodiments shown in FIGS. 4 to 9, quadrilateral grid structures in each grid structure unit A of the valve stent 100 are different. Each grid structure unit A includes one near-end grid structure 120, one middle supporting grid structure 130, and one far-end grid structure 140 sequentially arranged from the first axial end to the second axial end.

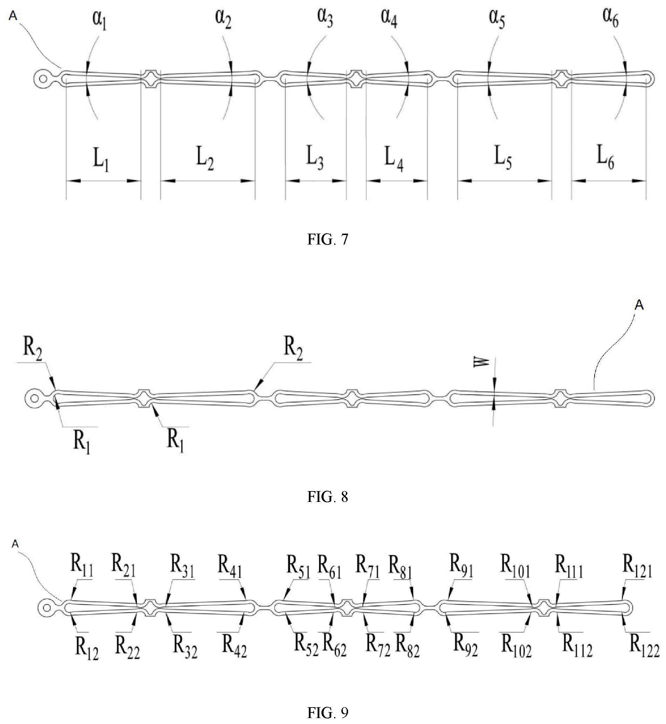

As shown in FIG. 7, L₁ is a length of the near-end grid structure 120 along an axial direction of the grid structure unit A between centers of arcuate connecting segments at two ends of two stent struts B (for distinguishing stent struts at different positions, the two stent struts are also represented as 121 in FIG. 6) adjacent to the first axial end; L₂ is a length of the near-end grid structure 120 along the axial direction of the grid structure unit A between centers of arcuate connecting segments at two ends of two stent struts B (for distinguishing stent struts at different positions, the two stent struts are also represented as 122 in FIG. 6) adjacent to the second axial end; L₃ is a length of the middle supporting grid structure 130 along the axial direction of the grid structure unit A between centers of arcuate connecting segments at two ends of two stent struts B (for distinguishing stent struts at different positions, the two stent struts are also represented as 131 in FIG. 6) adjacent to the first axial end; L₄ is a length of the middle supporting grid structure 130 along the axial direction of the grid structure unit A between centers of arcuate connecting segments at two ends of two stent struts B (for distinguishing stent struts at different positions, the two stent struts are also represented as 132 in FIG. 6) adjacent to the second axial end; L₅ is a length of the far-end grid structure 140 along the axial direction of the grid structure unit A between centers of arcuate connecting segments at two ends of two stent struts B (for distinguishing stent struts at different positions, the two stent struts are also represented as 141 in FIG. 6) adjacent to the first axial end; and L₆ is a length of the far-end grid structure 140 along the axial direction of the grid structure unit A between centers of arcuate connecting segments at two ends of two stent struts B (for distinguishing stent struts at different positions, the two stent struts are also represented as 142 in FIG. 6) adjacent to the second axial end.

As shown in FIG. 7, α₁ is an included angle of the near-end grid structure 120 formed by straight segments B₁ of two stent struts B (for distinguishing stent struts at different positions, the two stent struts are also represented as 121 in FIG. 6) that are arranged opposite each other in a circumferential direction of the grid structure unit A adjacent to the first axial end; α₂ is an included angle of the near-end grid structure 120 formed by straight segments B₁ of two stent struts B (for distinguishing stent struts at different positions, the two stent struts are also represented as 122 in FIG. 6) that are arranged opposite each other in the circumferential direction of the grid structure unit A adjacent to the second axial end; α₃ is an included angle of the middle supporting grid structure 130 formed by straight segments B₁ of two stent struts B (for distinguishing stent struts at different positions, the two stent struts are also represented as 131 in FIG. 6) that are arranged opposite each other in the circumferential direction of the grid structure unit A adjacent to the first axial end; α₄ is an included angle of the middle supporting grid structure 130 formed by straight segments B₁ of two stent struts B (for distinguishing stent struts at different positions, the two stent struts are also represented as 132 in FIG. 6) that are arranged opposite each other in the circumferential direction of the grid structure unit A adjacent to the second axial end; α₅ is an included angle of the far-end grid structure 140 formed by straight segments B₁ of two stent struts B (for distinguishing stent struts at different positions, the two stent struts are also represented as 141 in FIG. 6) that are arranged opposite each other in the circumferential direction of the grid structure unit A adjacent to first axial end; and α₆ is an included angle of the near-end grid structure 120 formed by straight segments B₁ of two stent struts B (for distinguishing stent struts at different positions, the two stent struts are also represented as 142 in FIG. 6) that are arranged opposite each other in the circumferential direction of the grid structure unit A adjacent to second axial end.

In this embodiment, for each quadrilateral grid structure, ${L}_{i} \times tan \frac{{α}_{i}}{2} = {L}_{j} \times tan \frac{{α}_{j}}{2}$
Lᵢ is a length of the quadrilateral grid structure along the axial direction of the valve stent 100 between the centers of the arcuate connecting segments at two ends of the stent strut B adjacent to the first axial end; Lⱼ is a length of the quadrilateral grid structure along the axial direction of the valve stent 100 between the centers of the arcuate connecting segments at two ends of the stent strut B adjacent to the second axial end; αᵢ is an included angle of the quadrilateral grid structure formed by straight segments B₁ of two stent struts B adjacent to the first axial end; and αⱼ is an included angle of the quadrilateral grid structure formed by straight segments B₁ of two stent struts B adjacent to the second axial end.

For example, in this embodiment, for the near-end grid structure 120, i is 1 and j is 2; for the middle supporting grid structure 130, i is 3 and j is 4; and for the far-end grid structure 140, i is 5 and j is 6. Taking the middle supporting grid structure 130 as an example, when L₃ is equal to L₄, α₃ is equal to α₄.

Through simulation of the valve stent 100 having the included angle between the straight segments B₁ of the two stent struts B that are arranged opposite each other in the circumferential direction of the grid structure unit A during a forming process, a loading and releasing process of implantation of the valve stent 100, and a fatigue failure process under repeated biomechanical stress, the following conclusions can be obtained based on the results of finite element analysis.

When the above included angle (straight segment cutting angle) in each quadrilateral grid structure of the grid structure unit A of the valve stent 100 between straight segments B₁ of the two stent struts B that are arranged circumferentially opposite each other is 12° or less, the stent struts B have a lower risk of fracture than when the included angle exceeds 12°.

Table 1 lists included angles of some exemplary valve stents 100 in the post-cutting state. The valve stent 100 has a stable overall structure, which satisfies clinical application requirements, and is safe and reliable during service in a patient.

**Table 1. Included angles of exemplary valve stents in the post-cutting state**

| Example | α₁=α₆(°) | α₂=α₅(°) | α₃=α₄(°) |
|---|---|---|---|
| Example 1 | 2.68 | 3.59 | 3.67 |
| Example 2 | 3.52 | 3.52 | 3.55 |
| Example 3 | 2.56 | 3.58 | 3.41 |
| Example 4 | 5.71 | 4.54 | 6.97 |
| Example 5 | 1.90 | 1.38 | 3.21 |
| Example 6 | 0.65 | 1.38 | 2.13 |
| Example 7 | 1.51 | 1.49 | 2.45 |

In addition, during processing and shape setting of the valve stent 100, a risk of fracture is likely to occur at a bent portion of a connection between adjacent far-end grid structures 140 of adjacent two grid structure units A of the valve stent 100. When the straight segment cutting angle of the far-end grid structure 140 of the valve stent 100 is in a range of 2.83° to 4.67°, a lower risk of fracture occurs during processing.

Within a working diameter range corresponding to a compression amount of 4 mm to 8 mm, as the straight segment cutting angle increases, a radial supporting stiffness of the valve stent within the working diameter gradually decreases, indicating that the straight segment cutting angle should not be excessively increased.

The fatigue analysis indicates that a risk of fatigue fracture is likely to occur at two ends of a connection between the far-end grid structures 140 of adjacent two grid structure units A of the valve stent 100. When the straight segment cutting angle is less than 4.67°, as the straight segment cutting angle increases, the fatigue safety factor of the valve stent 100 gradually increases.

When the straight segment cutting angle of the middle supporting grid structure 130 of the grid structure unit A of the valve stent 100 is within a range of 2.83° to 4.67°, the valve stent 100 exhibits optimal overall performance in processing, loading and release, and prevention of fatigue failure.

As shown in FIG. 8, in this embodiment, inner radii of arcuate connecting segments of quadrilateral grid structures of the grid structure unit A may be identical. A difference between an inner radius R₁ and an outer radius R₂ of each arcuate connecting segment is equal to one straight segment width W. The inner radius R₁ of the arcuate connecting segment may be, for example, 0.49 mm.

In an embodiment not shown, the inner radii of the arcuate connecting segments of the quadrilateral grid structures of the grid structure unit A may be different.

As shown in FIG. 9, the straight segment B₁ is connected to the arcuate connecting segment through an arcuate transition segment, and a bending direction of the arcuate transition segment is opposite to a bending direction of the arcuate connecting segment.

In some embodiments of the valve stent 100, inner radii of the arcuate transition segments of the quadrilateral grid structures of the grid structure unit A may be identical. In FIG. 9, the dimensions are defined as follows:
R₁₁ represents an outer radius of an arcuate transition segment that is of two stent struts B in the near-end grid structure 120 adjacent to the first axial end and that is adjacent to the first axial end; R₅₁ represents an outer radius of an arcuate transition segment that is of two stent struts B in the middle supporting grid structure 130 adjacent to the first axial end and that is adjacent to the first axial end; R₉₁ represents an outer radius of an arcuate transition segment that is of two stent struts B in the far-end grid structure 140 adjacent to the first axial end and that is adjacent to the first axial end;
R₁₂ represents an inner radius of an arcuate transition segment that is of two stent struts B in the near-end grid structure 120 adjacent to the first axial end and that is adjacent to the first axial end; R₅₂ represents an inner radius of an arcuate transition segment that is of two stent struts B in the quadrilateral grid structure of the middle supporting body adjacent to the first axial end and that is adjacent to the first axial end; R₉₂ represents an inner radius of an arcuate transition segment that is of two stent struts B in the far-end grid structure 140 adjacent to the first axial end and that is adjacent to the first axial end;
R₂₁ represents an inner radius of an arcuate transition segment that is of two stent struts B in the near-end grid structure 120 adjacent to the first axial end and that is adjacent to the second axial end; R₆₁ represents an inner radius of an arcuate transition segment that is of two stent struts B in the quadrilateral grid structure of the middle supporting body adjacent to the first axial end and that is adjacent to the second axial end; R₁₀₁ represents an inner radius of an arcuate transition segment that is of two stent struts B in the far-end grid structure 140 adjacent to the first axial end and that is adjacent to the second axial end;
R₂₂ represents an outer radius of an arcuate transition segment that is of two stent struts B in the near-end grid structure 120 adjacent to the first axial end and that is adjacent to the second axial end; R₆₂ represents an outer radius of an arcuate transition segment that is of two stent struts B in the quadrilateral grid structure of the middle supporting body adjacent to the first axial end and that is adjacent to the second axial end; R₁₀₂ represents an outer radius of an arcuate transition segment that is of two stent struts B in the far-end grid structure 140 adjacent to the first axial end and that is adjacent to the second axial end;
R₃₁ represents an inner radius of an arcuate transition segment that is of two stent struts B in the near-end grid structure 120 adjacent to the second axial end and that is adjacent to the first axial end; R₇₁ represents an inner radius of an arcuate transition segment that is of two stent struts B in the quadrilateral grid structure of the middle supporting body adjacent to the second axial end and that is adjacent to the first axial end; R₁₁₁ represents an inner radius of an arcuate transition segment that is of two stent struts B in the far-end grid structure 140 adjacent to the second axial end and that is adjacent to the first axial end;
R₃₂ represents an outer radius of an arcuate transition segment that is of two stent struts B in the near-end grid structure 120 adjacent to the second axial end and that is adjacent to the first axial end; R₇₂ represents an outer radius of an arcuate transition segment that is of two stent struts B in the quadrilateral grid structure of the middle supporting body adjacent to the second axial end and that is adjacent to the first axial end; R₁₁₂ represents an outer radius of an arcuate transition segment that is of two stent struts B in the far-end grid structure 140 adjacent to the second axial end and that is adjacent to the first axial end;
R₄₁ represents an outer radius of an arcuate transition segment that is of two stent struts B in the near-end grid structure 120 adjacent to the second axial end and that is adjacent to the second axial end; R₈₁ represents an outer radius of an arcuate transition segment that is of two stent struts B in the quadrilateral grid structure of the middle supporting body adjacent to the second axial end and that is adjacent to the second axial end; R₁₂₁ represents an outer radius of an arcuate transition segment that is of two stent struts B in the far-end grid structure 140 adjacent to the second axial end and that is adjacent to the second axial end;
R₄₂ respectively represent represents an inner radius of an arcuate transition segment that is of two stent struts B in the near-end grid structure 120 adjacent to the second axial end and that is adjacent to the second axial end; R₈₂ represents an inner radius of an arcuate transition segment that is of two stent struts B in the quadrilateral grid structure of the middle supporting body adjacent to the second axial end and that is adjacent to the second axial end; and R₁₂₂ represents an inner radius of an arcuate transition segment that is of two stent struts B in the far-end grid structure 140 adjacent to the second axial end and that is adjacent to the second axial end.

The inner radius and the outer radius of the arcuate transition segment differ by one straight segment width W. The inner radius of the arcuate transition segment may be, for example, 3.86 mm.

In an embodiment not shown, the dimensions indicated in FIG. 9 may be partially identical or may all be different.

As shown in FIGS. 4 to 9, in the valve stent 100 of this embodiment, the length of the near-end grid structure 120 along the axial direction of the valve stent 100 between the centers of the arcuate transition segments at two ends of the stent strut B adjacent to the first axial end is less than the length of the near-end grid structure 120 along the axial direction of the valve stent 100 between the centers of the arcuate transition segments at two ends of the stent strut B adjacent to the second axial end. The length of the far-end grid structure 140 along the axial direction of the valve stent 100 between the centers of the arcuate transition segments at two ends of the stent strut B adjacent to the first axial end is greater than the length of the far-end grid structure 140 along the axial direction of the valve stent 100 between the centers of the arcuate transition segments at two ends of the stent strut B adjacent to the second axial end.

For example, the length of the near-end grid structure 120 along the axial direction of the valve stent 100 between the centers of the arcuate transition segments at two ends of the stent strut B adjacent to the first axial end is 10.20 mm; the length of the near-end grid structure 120 along the axial direction of the valve stent 100 between the centers of the arcuate transition segments at two ends of the stent strut B adjacent to the second axial end is 11.85 mm; the length of the far-end grid structure 140 along the axial direction of the valve stent 100 between the centers of the arcuate transition segments at two ends of the stent strut B adjacent to the second axial end is 10.20 mm; and the length of the far-end grid structure 140 along the axial direction of the valve stent 100 between the centers of the arcuate transition segments at two ends of the stent strut B adjacent to the first axial end is 11.85 mm.

As shown in FIG. 10, in this embodiment, in the expanded state, an outer diameter D₂ at the first axial end (corresponding to the lower end in FIG. 10) and at the second axial end (corresponding to the upper end in FIG. 10) of the valve stent 100 is greater than an outer diameter D₁ at an axial middle portion, thereby forming a dumbbell-shaped structure in which the outer diameters at the two axial ends are greater than the outer diameter at the axial middle portion.

As shown in FIGS. 10 and 11, when the valve stent 100 is in the expanded state, the valve stent 100 transitions from the axial middle portion toward the first axial end sequentially through the first inwardly concave arcuate segment 1A and the first outwardly convex arcuate segment 1B, and transitions from the axial middle portion toward the second axial end sequentially through the second inwardly concave arcuate segment 1C and the second outwardly convex arcuate segment 1D.

A cross section of the first inwardly concave arcuate segment 1A passing through an axis of the valve stent 100 is a first inwardly concave arc having a radius R_{A} and recessed toward an inner side of the valve stent 100; a cross section of the first outwardly convex arcuate segment 1B passing through the axis of the valve stent 100 is a first outwardly convex arc having a radius R_{B} and protruding toward an outer side of the valve stent 100; a cross section of the second inwardly concave arcuate segment 1C passing through the axis of the valve stent 100 is a second inwardly concave arc having a radius R_{C} and recessed toward the inner side of the valve stent 100; and a cross section of the second outwardly convex arcuate segment 1D passing through the axis of the valve stent 100 is a second outwardly convex arc having a radius R_{D} and protruding toward the outer side of the valve stent 100.

R_{D} and R_{B} may be equal and may be, for example, 5 mm, 6 mm, 7 mm, 8 mm, 9 mm, or 10 mm.

R_{C} and R_{A} may be equal and may be, for example, 5 mm, 7 mm, 9 mm, 10 mm, 11 mm, or 13 mm.

In the expanded state, the valve stent 100 has a total height H₁, and the middle supporting grid structure 130 has a height H₂. Table 2 shows an outer shape dimension of the valve stent 100 in the expanded state.

**Table 2. Outer shape dimensions of the valve stent in the expanded state.**

| Example | Diameter at middle portion of valve stent D₁ mm | Diameter at two ends of valve stent D₂ mm | Total height H₁ mm | Height at middle portion H₂ mm | Applicable pulmonary artery and outflow tract diameter (mm) |
|---|---|---|---|---|---|
| Example 1 | 20.0±2.0 | 28.0±2.0 | 38.0±3.0 | 15.0±4.0 | 20.0-24.0 |
| Example 2 | 23.0±2.0 | 32.0±2.0 | 42.0±3.0 | 16.0±4.0 | 22.0-28.0 |
| Example 3 | 26.0±2.0 | 36.0±2.5 | 46.0±3.5 | 20.0±4.0 | 28.0-32.0 |
| Example 4 | 26.0±2.0 | 40.0±3.0 | 50.0±3.5 | 20.0±4.0 | 32.0-36.0 |
| Example 5 | 26.0±2.0 | 44.0±3.0 | 54.0±4.0 | 20.0±4.0 | 36.0-40.0 |
| Example 6 | 29.0±2.0 | 48.0±2.0 | 52.0±3.0 | 24.0±4.0 | 40.0-44.0 |
| Example 7 | 29.0±2.0 | 52.0±2.0 | 52.0±3.0 | 24.0±4.0 | 44.0-48.0 |

As shown in FIG. 12, the method for forming the valve stent according to the embodiment of the present disclosure includes the following steps.

A stent cutting step, including: cutting metal tubing to form a blank having the same structure as the valve stent 100 in a post-cutting state, and causing the two stent struts B that are arranged opposite each other in the circumferential direction of the valve stent 100 in each quadrilateral grid structure of the blank to gradually diverge from each other from the axial middle portion toward the axial end portion of the quadrilateral grid structure to form an included angle, where the included angle of the middle supporting grid structure 130 formed by the straight segments B₁ of the two stent struts B that are arranged opposite each other in the circumferential direction of the valve stent 100 is less than or equal to 12°. The blank in the post-cutting state is formed by laser cutting. A power of a laser cutting machine is selected to completely cut through a single-layer nickel-titanium alloy tubing. Argon gas is used for shielding, and a strike point is located at a middle portion of the straight segment B₁. Since no stress concentration exists at the middle portion of the straight segment B₁, and the strike point generally produces a relatively large cutting spot with dimensional deviation, locating the strike point at the middle portion of the straight segment B₁ helps prevent fracture of the stent strut B.

A stent grinding step, including grinding the blank with emery and purified water for 5 min to 20 min. Grinding the valve stent 100 can remove slag adhering to a wall surface of the valve stent 100.

A stent pre-shaping step, including performing multi-step pre-shaping on the blank in the post-cutting state and performing annealing after each pre-shaping step to form a blank in an expanded state. The multi-step pre-shape setting includes 2 to 6 pre-shaping steps. A maximum strain in each pre-shaping step is less than 8%. The annealing treatment is performed at an annealing temperature of 400 °C to 500 °C, with a holding time of 2 min to 21 min, and a cooling mode of water cooling.

A stent shaping step, including fixing the blank in the expanded state in a shaping mold; and performing annealing shaping treatment on the blank fixed in the shaping mold. The annealing shaping treatment is performed at an annealing temperature of 500 °C to 550 °C, with a holding time of 2 min to 30 min, and a cooling mode of water cooling.

A stent sandblasting step, including sandblasting the valve stent 100 in the expanded state.

A stent polishing step, including polishing the sandblasted valve stent 100.

A stent cleaning step, which may include a stent rough-cleaning step and a stent fine-cleaning step, where the stent rough-cleaning step may include rinsing with water, and the stent fine-cleaning step may include ultrasonic cleaning.

A radiopaque marker fixing step, including fixedly mounting a radiopaque material in the fixing groove 102.

As shown in FIG. 12, this embodiment further provides a method for forming a artificial heart valve. This processing method includes the following steps.

A pericardium pre-shaping step, including pericardium harvesting, pericardium rinsing, pericardium fixation treatment, pericardium screening, and the like. The pericardium may be porcine pericardium.

A valve leaflet and sealing membrane manufacturing step.

A pericardium biological deloading step.

A valve suturing step, including suturing the valve leaflets and the sealing membrane onto the valve stent 100 to form the artificial heart valve.

A chemical treatment step, which is a key process in processing the artificial heart valve and is used for treating tissue to inhibit calcification after implantation. The chemical treatment step includes heating a glutaraldehyde solution to a first temperature. The first temperature may be 15 °C to 90 °C. More preferably, the glutaraldehyde solution is heated to the first temperature between 55 °C and 75 °C and maintained for 0.5 day to 10 days. Then, before contacting bioprosthetic tissue, the temperature of the glutaraldehyde solution is adjusted to a second temperature. The second temperature may be 30 °C to 70 °C, preferably 45 °C to 55 °C. A tissue treatment time may range from 0.1 day to 15 days, more preferably from about 0.1 day to about 12 days. For example, at a temperature of about 50 °C, a preferred treatment time is about 5 days to 10 days, and a more preferred treatment time is about 7 days.

A fine-cleaning step.

A sterilization step.

It should be understood by those skilled in the art that, in the foregoing methods of the specific embodiments, the order in which the steps are described does not imply a strict execution sequence or impose any limitation on the implementation process. The specific execution sequence of the steps should be determined according to the respective functions and possible inherent logic thereof.

The foregoing descriptions of the embodiments emphasize the differences among the embodiments. Identical or similar features among the embodiments may be referenced to one another. For the sake of brevity, details are not repeated herein.

Finally, it should be noted that the foregoing embodiments are merely intended to illustrate the technical solutions of the present disclosure rather than to limit the present disclosure. Although the present disclosure has been described in detail with reference to preferred embodiments, those of ordinary skill in the art should understand that modifications may still be made to the specific embodiments of the present disclosure, or equivalent substitutions may be made for some technical features, without departing from the scope of the technical solutions for which protection is sought by the present disclosure.

## Claims

1. A valve stent, wherein the valve stent (100) is formed by cutting metal tubing and has a post-cutting state and an expanded state, the valve stent (100) comprises a plurality of grid structure units (A) distributed along a circumferential direction of the valve stent (100), and the plurality of grid structure units (A) extend along an axial direction of the valve stent (100); each grid structure unit comprises a plurality of quadrilateral grid structure, the plurality of quadrilateral grid structures comprise at least one near-end grid structure (120), at least one middle supporting grid structure (130), and at least one far-end grid structure (140) sequentially arranged along the axial direction of the valve stent (100) from a first axial end to a second axial end; each quadrilateral grid structure comprises four stent struts (B) connected end to end in sequence, each stent strut (B) comprises a straight segment (B₁) and a connecting segment (B₂), the straight segments (B₁) of the stent struts (B) form a grid body of the quadrilateral grid structure, the connecting segments (B₂) of the stent struts (B) form corner portions of the quadrilateral grid structure, adjacent stent struts (B) of each quadrilateral grid structure are connected through the connecting segment (B₂), and corner portions at an axial middle portion of a corresponding quadrilateral grid structure of adjacent grid structure units (A) are connected, and in the post-cutting state, straight segments (B₁) of two stent struts (B) that are arranged opposite each other in the circumferential direction of the valve stent (100) in each quadrilateral grid structure gradually diverge from each other from the axial middle portion toward an axial end portion of the corresponding quadrilateral grid structure to form an included angle, wherein
an included angle of the middle supporting grid structure (130) formed by the straight segments (B₁) of the two stent struts (B) that are arranged opposite each other in the circumferential direction of the valve stent (100) is less than or equal to 12°.

2. The valve stent according to claim 1, wherein the plurality of quadrilateral grid structures comprise two middle supporting grid structures (130), and the included angle of each middle supporting grid structure (130) formed by the straight segments (B₁) of the two stent struts (B) that are arranged opposite each other in the circumferential direction of the valve stent (100) is less than 11.74°; or
the plurality of quadrilateral grid structures comprise one middle supporting grid structure (130), and the included angle of the middle supporting grid structure (130) formed by the straight segments (B₁) of the two stent struts (B) that are arranged opposite each other in the circumferential direction of the valve stent (100) is less than 6.14°.

3. The valve stent according to claim 2, wherein
the plurality of quadrilateral grid structures comprise two middle supporting grid structures, and the included angle of each middle supporting grid structure defined by the straight segments of the every two stent struts that are opposite each other in the circumferential direction of the valve stent is 5.0° to 11.0°; or the plurality of quadrilateral grid structures comprise one middle supporting grid structure, and the included angle of the middle supporting grid structure defined by the straight segments of the every two stent struts that are opposite each other in the circumferential direction of the valve stent is 2.5° to 5.5°.

4. The valve stent according to any one of claims 1 to 3, wherein
in the post-cutting state, an included angle of the middle supporting grid structure (130) formed by the straight segments (B₁) of the two stent struts (B) adjacent to the first axial end is equal to or different from an included angle of the middle supporting grid structure (130) formed by the straight segments (B₁) of the two stent struts (B) adjacent to the second axial end.

5. The valve stent according to any one of claims 1 to 4, wherein
the connecting segment (B₂) of the stent strut (B) comprises two curved connecting segments respectively provided at two ends of the straight segment (B₁), and two adjacent stent struts (B) in the quadrilateral grid structure are connected through the curved connecting segments;
a curved connecting segment of the stent strut (B) adjacent to an axial end portion of a quadrilateral grid structure in which the stent strut (B) is located is curved toward an inner side of the quadrilateral grid structure; and
a curved connecting segment of the stent strut (B) adjacent to an axial middle portion of the quadrilateral grid structure in which the stent strut (B) is located is curved toward an outer side of the quadrilateral grid structure.

6. The valve stent according to claim 5, wherein
the curved connecting segment is an arcuate connecting segment, and a ratio of a length of the straight segment to an inner radius of the arcuate connecting segment is in a range of 4 to 30.

7. The valve stent according to claim 5 or 6, wherein the straight segment (B₁) is connected to the curved connecting segment through a curved transition segment (B₃), and a bending direction of the curved transition segment is opposite to a bending direction of the curved connecting segment.

8. The valve stent according to claim 7, wherein
the curved transition segment (B₃) is an arcuate transition segment, and a ratio of an inner radius of the arcuate transition segment to a length of the straight segment is in a range of 0.25 to 0.75.

9. The valve stent according to any one of claims 5 to 8, wherein the curved connecting segment is an arcuate connecting segment;
a length of the near-end grid structure (120) along the axial direction of the valve stent (100) between centers of arcuate connecting segments at two ends of a stent strut (B) adjacent to the first axial end is equal to a length of the near-end grid structure (120) along the axial direction of the valve stent (100) between the centers of the arcuate connecting segments at two ends of the stent strut (B) adjacent to the second axial end;
and/or, a length of the far-end grid structure (140) along the axial direction of the valve stent (100) between the centers of the arcuate connecting segments at two ends of the stent strut (B) adjacent to the first axial end is equal to or different from a length of the far-end grid structure (140) along the axial direction of the valve stent (100) between the centers of the arcuate connecting segments at two ends of the stent strut (B) adjacent to the second axial end;
and/or, a length of the middle supporting grid structure (130) along the axial direction of the valve stent (100) between the centers of the arcuate connecting segments at two ends of the stent strut (B) adjacent to the first axial end is equal to or different from a length of the middle supporting grid structure (130) along the axial direction of the valve stent (100) between the centers of the arcuate connecting segments at two ends of the stent strut (B) adjacent to the second axial end.

10. The valve stent according to claim 9, wherein
the length of the near-end grid structure (120) along the axial direction of the valve stent (100) between the centers of the arcuate connecting segments at two ends of the stent strut (B) adjacent to the first axial end is less than the length of the near-end grid structure (120) along the axial direction of the valve stent (100) between the centers of the arcuate connecting segments at two ends of the stent strut (B) adjacent to the second axial end;
and/or,
the length of the far-end grid structure (140) along the axial direction of the valve stent (100) between the centers of the arcuate connecting segments at two ends of the stent strut (B) adjacent to the first axial end is greater than the length of the far-end grid structure (140) along the axial direction of the valve stent (100) between the centers of the arcuate connecting segments at two ends of the stent strut (B) adjacent to the second axial end.

11. The valve stent according to claim 9 or 10, wherein
a ratio of the length of the near-end grid structure (120) along the axial direction of the valve stent (100) between the centers of the arcuate connecting segments at two ends of the stent strut (B) adjacent to the first axial end and the length of the near-end grid structure (120) along the axial direction of the valve stent (100) between the centers of the arcuate connecting segments at two ends of the stent strut (B) adjacent to the second axial end is 0.65 to 1.00; and/or
a ratio of the length of the far-end grid structure (140) along the axial direction of the valve stent (100) between the centers of the arcuate connecting segments at two ends of the stent strut (B) adjacent to the second axial end and the length of the far-end grid structure (140) along the axial direction of the valve stent (100) between the centers of the arcuate connecting segments at two ends of the stent strut (B) adjacent to the first axial end is 0.65 to 1.00.

12. The valve stent according to any one of claims 5 to 11, wherein
the curved connecting segment is an arcuate connecting segment;
the plurality of quadrilateral grid structures comprise one near-end grid structure (120), one middle supporting grid structure (130), and one far-end grid structure (140) sequentially arranged along the axial direction of the valve stent (100) from the first axial end to the second axial end;
a length of the middle supporting grid structure (130) along the axial direction of the valve stent (100) between centers of arcuate connecting segments at two ends of each stent strut (B) is in a range of 3.05 mm to 8.22 mm; and/or
a length of the near-end grid structure (120) along the axial direction of the valve stent (100) between the centers of the arcuate connecting segments at two ends of each stent strut (B) is in a range of 3.55 mm to 12.50 mm; and/or
a length of the far-end grid structure (140) along the axial direction of the valve stent (100) between the centers of the arcuate connecting segments at two ends of each stent strut (B) is in a range of 3.55 mm to 12.50 mm.

13. The valve stent according to any one of claims 5 to 12, wherein
the curved connecting segment is an arcuate connecting segment;
for each grid structure unit, ${L}_{i} \times tan \frac{{α}_{i}}{2} = {L}_{j} \times tan \frac{{α}_{j}}{2} ;$
wherein
Lᵢ is a length of the quadrilateral grid structure along the axial direction of the valve stent (100) between centers of arcuate connecting segments at two ends of a stent strut (B) adjacent to the first axial end;
Lⱼ is a length of the quadrilateral grid structure along the axial direction of the valve stent (100) between the centers of the arcuate connecting segments at two ends of the stent strut (B) adjacent to the second axial end;
αᵢ is an included angle of the quadrilateral grid structure formed by the straight segments (B₁) of the two stent struts (B) adjacent to the first axial end; and
αⱼ is an included angle of the quadrilateral grid structure formed by the straight segments (B₁) of the two stent struts (B) adjacent to the second axial end.

14. The valve stent according to any one of claims 1 to 13, wherein
the valve stent (100) further comprises a plurality of connecting rods (150);
the near-end grid structure (120) and the middle supporting grid structure (130) of each grid structure unit are connected through one connecting rod (150); or, along the circumferential direction of the valve stent (100), one grid structure unit in which the near-end grid structure (120) and the middle supporting grid structure (130) are connected through one connecting rod (150) is provided for every one or two grid structure units (A) in which the near-end grid structure (120) is not connected to the middle supporting grid structure (130) through the connecting rod (150); and/or
the far-end grid structure (140) and the middle supporting grid structure (130) of each grid structure unit are connected through one connecting rod (150); or, along the circumferential direction of the valve stent (100), one grid structure unit in which the far-end grid structure (140) and the middle supporting grid structure (130) are connected through one connecting rod (150) is provided for every one or two grid structure units (A) in which the far-end grid structure (140) is not connected to the middle supporting grid structure (130) through the connecting rod (150).

15. The valve stent according to any one of claims 1 to 14, wherein
in the expanded state, an outer diameter D₂ at the first axial end and the second axial end of the valve stent (100) is greater than an outer diameter D₁ at the axial middle portion.

16. The valve stent according to claim 15, wherein
in the expanded state, the outer diameter D₁ at the axial middle portion of the valve stent (100) is in a range of 18.0 mm to 32.0 mm, and the outer diameter D₂ at the first axial end and the second axial end of the valve stent (100) is in a range of 20.0 mm to 54.0 mm.

17. The valve stent according to claim 15 or 16, wherein in the expanded state, a total height H₁ of the valve stent (100) is in a range of 30.0 mm to 60.0 mm, and a height H₂ of the middle supporting grid structure (130) is in a range of 12.0 mm to 30.0 mm.

18. The valve stent according to any one of claims 15 to 17, wherein in the expanded state, the valve stent (100) transitions from the axial middle portion toward the first axial end sequentially through a first inwardly concave arcuate segment (1A) and a first outwardly convex arcuate segment (1B), and transitions from the axial middle portion toward the second axial end sequentially through a second inwardly concave arcuate segment (1C) and a second outwardly convex arcuate segment (1D), wherein a cross section of the first inwardly concave arcuate segment (1A) passing through an axis of the valve stent (100) is a first inwardly concave arc having a radius R_{A} and recessed toward an inner side of the valve stent (100); a cross section of the first outwardly convex arcuate segment (1B) passing through the axis of the valve stent (100) is a first outwardly convex arc having a radius R_{B} and protruding toward an outer side of the valve stent (100); a cross section of the second inwardly concave arcuate segment (1C) passing through the axis of the valve stent (100) is a second inwardly concave arc having a radius R_{C} and recessed toward the inner side of the valve stent (100); and a cross section of the second outwardly convex arcuate segment (1D) passing through the axis of the valve stent (100) is a second outwardly convex arc having a radius R_{D} and protruding toward the outer side of the valve stent (100); wherein R_{A} and R_{C} each range from 3.0 mm to 15.0 mm, and R_{B} and R_{D} each range from 3.0 mm to 15.0 mm.

19. A the valve stent according to any one of claims 1 to 18, comprising:
a stent cutting step, comprising: cutting metal tubing to form a blank having the same structure as the valve stent (100) in a post-cutting state, and causing the two stent struts (B) that are arranged opposite each other in the circumferential direction of the valve stent (100) in each quadrilateral grid structure of the blank to gradually diverge from each other from the axial middle portion toward the axial end portion of the quadrilateral grid structure to form an included angle, wherein the included angle of the middle supporting grid structure (130) formed by the straight segments (B₁) of the two stent struts (B) that are arranged opposite each other in the circumferential direction of the valve stent (100) is less than or equal to 12°;
a stent pre-shaping step, comprising: performing multi-step pre-shaping on the blank in the post-cutting state and performing annealing after each pre-shaping step to form a blank in an expanded state; and
a stent shaping step, comprising: shaping the blank in the expanded state after the stent pre-shaping step by using a shaping mold to form the valve stent (100) in the expanded state.

20. The method for forming the valve stent according to claim 19, wherein
in the stent pre-shaping step, the multi-step pre-shaping comprises 2 to 6 pre-shaping steps; and/or
a maximum strain in each pre-shaping step is less than 12%; and/or
the annealing treatment is performed at an annealing temperature of 400 °C to 500 °C, with a holding time of 2 min to 21 min, and a cooling mode of water cooling; and/or
the stent shaping step comprises: fixing the blank in the expanded state within the shaping mold; and performing annealing shaping treatment on the blank fixed within the shaping mold, wherein the annealing shaping treatment is performed at an annealing temperature of 500 °C to 550 °C, with a holding time of 2 min to 30 min, and a cooling mode of water cooling.

21. An artificial heart valve, comprising a valve stent,wherein the valve stent (100) is the valve stent (100) according to any one of claims 1 to 18.
